# FASCICULE DE BREVET EUROPEEN

(11) **EP 1 006 931 B1**
(45) Date de publication et mention de la délivrance du brevet: **10.03.2004**
(21) Numéro de dépôt: 96943171.7
(22) Date de dépôt: 20.12.1996
(51) Int. Cl.: A61C 13/00, G05B 19/42

(54) **PROCEDE POUR REALISER UNE PROTHESE DENTAIRE**
VERFAHREN ZUR HERSTELLUNG EINER ZAHNPROTHESE
METHOD FOR PRODUCING A DENTAL PROSTHESIS

(43) Date de publication de la demande: 14.06.2000
(73) Titulaire: Cynovad Inc., West Montréal, Québec H3B 4W8 (CA)
(72) Inventeur: PEROT, Jean-Marc, F-38200 Vienne (FR); DIVET, Michel, F-69007 Lyon (FR); ROLET, Guy, F-69004 Lyon (FR)
(74) Mandataire: Maureau, Philippe
(86) Numéro de dépôt international: PCT/FR1996/002055
(87) Numéro de publication internationale: WO 1998/027890

(56) Documents cités:
- EP-A- 0 311 214
- EP-A- 0 580 565
- EP-A- 0 643 948
- WO-A-96/10371
- FR-A- 2 536 654
- US-A- 5 382 164

## Description

La présente invention est relative au domaine dentaire et elle concerne, plus particulièrement, les prothèses du type fixées ou conjointes, telles que bridges, couronnes, faux moignons, implants ou amovoinamovibles.

Pour des raisons économiques, il a été prévu de réaliser les prothèses dentaires le plus souvent en un alliage métallique précieux, semi précieux ou non précieux, tel qu'en alliage nickel-chrome ou en alliage à base de cobalt.

Pour des raisons esthétiques évidentes, la constitution d'une telle prothèse, qui était uniquement de nature métallique, a évolué pour faire intervenir la fabrication d'une chape toujours de structure métallique mais recouverte d'une coiffe en céramique. La chape présente un profil adapté à la zone d'implantation du corps humain. Le profil de la chape est réalisé à partir d'une empreinte de la zone d'implantation.

Il est clair que la fabrication de telles prothèses présente un certain nombre d'inconvénients en raison, notamment, de l'importance du travail en bouche, des délais de réalisation importants, du coût élevé de main d'oeuvre, d'une distorsion entre l'appréciation du dentiste et celle du prothésiste et d'une nécessité de plusieurs interventions en bouche, qui sont longues et pénibles pour le patient.

Pour tenter de remédier aux inconvénients énoncés ci-dessus, il a été imaginé dans l'état de la technique, des solutions visant à tenter d'automatiser la fabrication de telles prothèses.

Le document EP-A-643 948 concerne un procédé de réalisation d'une prothèse dentaire comprenant une chape et une coiffe. La chape comprend une surface interne qui est une duplication de la forme du moignon à équiper, et une surface externe qui est une dilatation mathématique de la surface du moignon.

Le document FR-A-2 536 654 a ainsi décrit un procédé de réalisation d'une prothèse dentaire consistant, dans un premier temps, à saisir la forme de la zone d'implantation, par empreinte, micro-palpage ou empreinte optique puis, dans un deuxième temps, à usiner, de façon automatique, la pièce prothétique en tenant compte des données saisies et des informations apportées par un logiciel de traitement. Selon une première variante de réalisation, il est prévu d'usiner, d'une part, la face interne de la chape à la forme de la zone d'implantation et la face externe de la coiffe en fonction de l'enveloppe et de l'occlusion. La chape et la coiffe sont sélectionnées à partir d'un stock de coiffes et de chapes qui sont appariées de manière que la face externe de la chape possède le même profil que la face interne de la coiffe.

Ce document décrit, notamment, une deuxième variante de réalisation consistant, à partir d'une saisie des formes effectuées en bouche, à réaliser, dans un bloc métallique, l'usinage des faces interne et externe de la chape métallique, à faire une nouvelle saisie de la faxe externe de la couronne métallique, à réaliser en fonction de celle-ci et à partir d'un bloc céramique, l'usinage de la face interne de la coiffe, puis, en fonction de l'enveloppe et de l'occlusion, l'usinage de la face externe de celle-ci avant de procéder à l'assemblage de la chape métallique avec la coiffe céramique.

Si un tel document décrit un procédé de réalisation automatique d'une prothèse dentaire, il apparaît que la mise en oeuvre pratique d'un tel procédé présente des inconvénients. Il doit tout d'abord être constaté qu'un tel procédé impose, selon sa première variante de réalisation, de disposer d'un stock de coiffes et de chapes à apparier, dont le nombre doit être relativement important pour tenter de couvrir l'ensemble des configurations de prothèses à réaliser. Il s'avère, en pratique, impossible de disposer en stock, des prothèses s'adaptant à toutes les morphologies des zones d'implantation. Par ailleurs, la seconde variante de réalisation nécessite la mise en oeuvre de plusieurs opérations de fabrication.

Plus fondamentalement, il doit être constaté que la chape métallique est généralement réalisée de façon homothétique à la zone d'implantation, tandis que la coiffe présente une surface extérieure adaptée à l'enveloppe de l'environnement buccal. La Déposante a constaté qu'une telle démarche conduisait, dans certains cas, à la réalisation de coiffes présentant des zones localisées de moindre résistance, dont la présence affecte indubitablement la fiabilité des prothèses ainsi réalisées. Egalement, cette technique conduit parfois à l'obtention de prothèses inesthétiques, en raison de l'impossibilité à réaliser, au moins localement, la coiffe en céramique. Il apparaît donc le besoin de disposer d'une méthode de fabrication adaptée, d'une part, pour tenir compte de la situation clinique de la dent à appareiller et, d'autre part, pour respecter les règles de de la dent à appareiller et, d'autre part, pour respecter les règles de l'esthétique et les critères de tenue mécanique que doivent respecter les coiffes céramiques pour offrir une fiabilité dans le temps.

L'objet de l'invention vise donc à proposer un procédé pour réaliser une prothèse dentaire, conçu pour permettre de définir le profil de la chape et d'une coiffe en tenant compte, respectivement, de la zone d'implantation et de l'environnement dé la prothèse tout en garantissant une optimisation du profil de la chape pour garantir le respect des critères de tenue mécanique et d'esthétique de la coiffe.

Pour atteindre cet objectif, le procédé selon l'invention est adapté pour permettre la réalisation d'une prothèse dentaire comportant au moins, d'une part, une chape prothétique destinée à être adaptée sur une zone d'implantation du corps humain et, d'autre part, une coiffe prothétique portée par la chape.

Selon l'invention, le procédé consistant à :
- réaliser *par moulage une empreinte (6) de la zone d'implantation et établir à partir de l'empreinte moulée (6)* une représentation tridimensionnelle numérisée (R,),
- définir une représentation tridimensionnelle (R_{1c}) de la surface interne de la prothèse, correspondant à la surface interne de la chape définie à partir de la représentation tridimensionnelle numérisée (R₁) de la zone d'implantation, en tenant compte des règles liées à l'insertion et au scellement d'une prothèse,
- définir une représentation tridimensionnelle numérisée (R₂) de la surface externe de la prothèse, à partir des paramètres environnementaux de la prothèse, en tenant compte des contraintes résultant de la surface interne (R_{1C}) de la prothèse, constituée par la surface interne de la chape,
*est caractérisé en ce qu'il consiste*
- à définir une représentation tridimensionnelle numérisée (R₃) de la surface externe de la chape, à partir de la surface externe (R₂) de la prothèse, en tenant compte de la surface interne (R_{1c}) de la prothèse, des critères de tenue mécanique, notamment d'épaisseur, et des critères esthétiques.

Le procédé selon l'invention présente donc l'avantage de permettre la définition d'une chape en tenant compte du profil anatomique de la zone d'implantation, de la surface externe de la prothèse et des critères de réalisation de la coiffe, en vue d'obtenir une prothèse fiable dans le temps. L'objet de l'invention présente également l'avantage de permettre de déterminer, sans nécessiter la fabrication d'une prothèse et à partir des informations cliniques, s'il est possible de réaliser la chape et la coiffe dans des conditions acceptables de fiabilité et d'esthétique. Un tel procédé offre ainsi la possibilité de choisir et d'adapter le profil de la chape en fonction des conditions de réalisation de la coiffe.

Diverses autres caractéristiques ressortent de la description faite ci-dessous en référence aux dessins annexés qui montrent, à titre d'exemples non limitatifs, des formes de réalisation et de mise en oeuvre de l'objet de l'invention :
La figure 1 est une vue schématique illustrant l'adaptation d'une prothèse sur une dent à appareiller ;
Les figures 2 à 4 sont des vues schématiques montrant chacune une étape caractéristique du procédé selon l'invention ;
La figure 5 est une vue montrant en perspective l'étape du procédé réalisée à la figure 4 ;
Les figures 6 et 7 sont des vues en coupe et en perspective illustrant une autre étape du procédé selon l'invention ;
La figure 8 est une perspective montrant un exemple de réalisation d'une représentation d'une prothèse à réaliser ;
La figure 9 est une vue en coupe illustrant une autre étape du procédé selon l'invention ;
Les figures 10 et 11 illustrent des exemples de réalisation des éléments d'une prothèse, obtenus selon le procédé selon l'invention ;
La figure 12 est une coupe montrant un avantage du procédé selon l'invention.

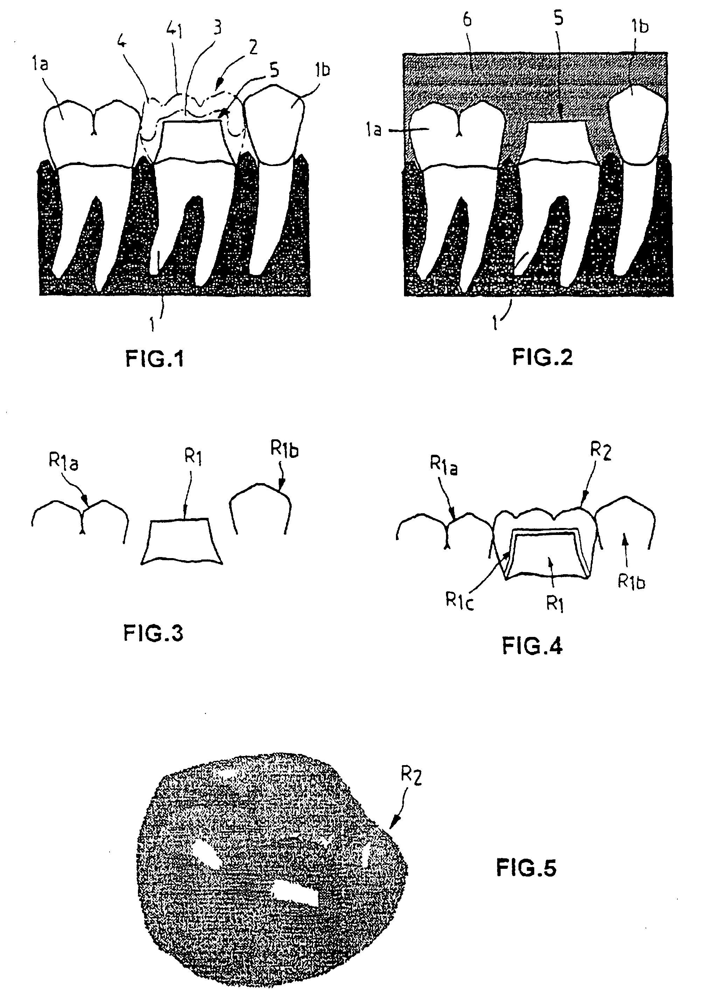

La figure 1 montre un exemple d'une dent à appareiller 1 par l'intermédiaire d'une prothèse 2 conforme à l'invention, comportant une chape prothétique d'adaptation 3 sur laquelle est montée une coiffe prothétique 4.

Pour exécuter une prothèse dentaire, du type de la figure 1, il est procédé classiquement, à la préparation de la dent à appareiller 1. Dans l'exemple illustré, la dent à appareiller 1 présente une zone d'implantation 5 pour la prothèse 2, constituée, dans l'exemple illustré, par un moignon. Bien entendu, il est clair que la partie du corps humain servant de zone d'implantation 5, peut présenter une forme différente, telle que notamment une cavité, voire une absence de moignon (pontique).

Tel que cela apparaît plus précisément à la figure 2, le procédé selon l'invention consiste à réaliser, par l'intermédiaire d'un moulage 6, une empreinte d'au moins la zone d'implantation 5. Le moule 6 est réalisé de toutes façons connues par l'homme du métier, en vue d'obtenir au moins un profil représentatif de la zone d'implantation 5, à savoir le moignon dans l'exemple illustré. A côté de la connaissance de la situation clinique liée spécifiquement à la zone d'implantation, il peut être prévu également de recueillir toutes les informations cliniques relatives à la réalisation de la prothèse, telles que celles relatives, par exemple, à la position des dents adjacentes 1a, 1b et/ou des dents antagonistes en position d'occlusion statique et/ou dynamique. Cette étape du procédé vise donc à recueillir, par une ou plusieurs opérations de moulage et d'éventuelles mesures cliniques et/ou morphologiques (cinématique mandibulaire, ligne du sourire, ... ), les informations relatives à l'implantation et à l'intégration de la prothèse à l'intérieur de la bouche du patient.

A partir de l'empreinte moulée 6, le procédé selon l'invention prévoit d'établir une représentation tridimensionnelle numérisée R, d'au moins de la zone d'implantation 5. La représentation numérisée R₁, de la zone d'implantation, qui est illustrée à la figure 3, est obtenue à partir d'un capteur, de préférence, de nature optique permettant de prendre des mesures de l'empreinte moulée 6, en vue d'obtenir la définition, en trois dimensions, de la zone d'implantation 5. Il doit être considéré que la prise des mesures peut être effectuée directement sur l'empreinte négative réalisée par le moule 6 de profil complémentaire de celui du moignon, ou sur un moule positif reproduisant le moignon et obtenu à partir du moule négatif 6. Le capteur utilisé transmet les mesures, après conversion, a un ordinateur qui assure l'enregistrement des données tridimensionnelles numériques de caractérisation de la zone d'implantation 5. L'ordinateur, qui est équipé d'un écran, est adapté pour assurer la visualisation d'une représentation tridimensionnelle R₁, de la zone d'implantation 5. Dans le cas où des informations cliniques sont recueillies sur l'environnement de la zone d'implantation 5, il est prévu d'en assurer une représentation numérisée, telle que par exemple R₁ₐ, R_{1b} pour les dents adjacentes et/ou antagonistes.

Le procédé selon l'invention consiste, ensuite, à définir une représentation tridimensionnelle numérisée R_{1c} de la surface interne de la prothèse définie à partir de la représentation numérisée R₁ de la zone d'implantation, en tenant compte des règles liées à l'insertion et au scellement d'une prothèse. Ainsi, il peut être prévu de laisser subsister, notamment, un espace entre la représentation numérisée R₁ de la zone d'implantation et la représentation numérisée R_{1c} de la surface interne de la prothèse pour la mise en place du ciment de scellement,

Le procédé selon l'invention consiste, ensuite, à définir une représentation tridimensionnelle numérisée R₂ de la surface externe de la prothèse, à partir de paramètres environnementaux de la prothèse et de la représentation tridimensionnelle numérisée R_{1c} de la surface interne de la prothèse. Tel que cela ressort plus précisément des figures 4 et 5, la représentation numérisée R₂ permet de visualiser la surface externe de la prothèse par rapport à la représentation numérisée R₁ de la zone d'implantation 5 et, éventuellement, des représentations des dents adjacentes et/ou antagonistes, telles que, par exemple, R₁ₐ, R_{1b}, La surface externe de la prothèse est donc déterminée en partant des éventuelles mesures réalisées sur l'empreinte moulée et d'une morphologie statistiquement représentative ou acceptable de la prothèse à réaliser, sur laquelle sont appliquées des lois de déformation fondées sur des critères cliniques acceptés par l'homme de l'art. Par exemple, il peut être prévu d'utiliser les critères tels que définis dans les cours d'anatomie dentaire. Il est à noter que le volume compris entre la surface R_{1c} et la surface R₂ est représentatif du volume total de la prothèse.

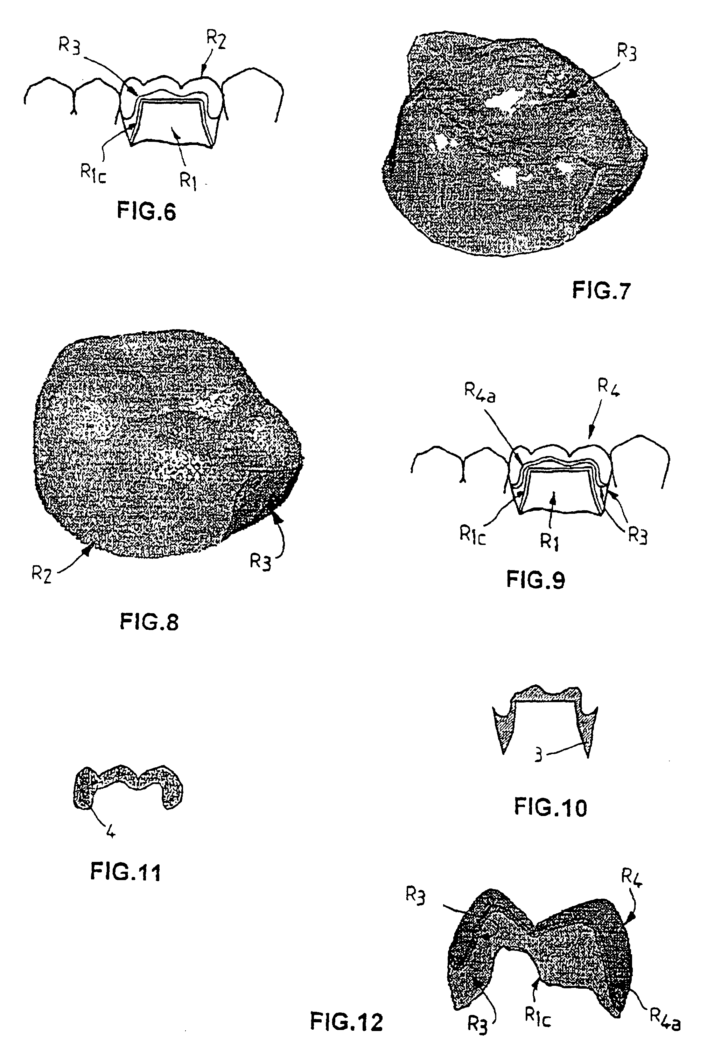

L'objet de l'invention consiste, ensuite, à déterminer une représentation tridimensionnelle numérisée R₃ de la surface externe de la chape prothétique 3, à partir de la représentation numérisée R_{1c}, de la surface interne de la prothèse et de la représentation R₂ de la surface externe de la prothèse. La représentation numérisée R₃ de la surface externe de la chape, dont un exemple est illustré par les figures 6 et 7, est réalisée en tenant compte des critères devant être respectés pour la définition de la coiffe 4 qui correspond au volume compris entre les représentations R₃ et R₂. Pour la définition de la coiffe, les critères à respecter sont, par exemple, les règles liées à l'esthétique ou à la forme et à l'épaisseur minimum et/ou maximum que la coiffe doit présenter en fonction de la nature du matériau utilisé. Le respect de ces critères permet d'éviter la présence de zones fragiles, en vue d'obtenir une prothèse fiable dans le temps. Le procédé selon l'invention présente également l'avantage de savoir si, avant de procéder à la fabrication de la prothèse, il est possible de réaliser une prothèse respectant les exigences anatomiques de l'environnement buccal et les contraintes mécaniques et esthétiques liées à la réalisation de la prothèse. Ainsi, comme illustré à la figure 8, il peut être prévu d'effectuer une représentation numérisée de la prothèse 2 à partir des représentations numérisées R₂, R₃, respectivement de la surface externe de la prothèse et de la surface externe de la chape.

Bien entendu, il doit être considéré que la représentation numérisée R₃ de la surface externe de la chape est assurée par des moyens de programmation appropriés tenant compte des représentations numérisées R_{1c} R₂ respectivement de la surface interne de la prothèse et de la surface externe de la prothèse et des critères de fabrication de la coiffe préalablement enregistrés. Il est à noter que le volume compris entre la surface R_{1c} et la surface R₃ est représentatif du volume total de la chape.

Selon une autre caractéristique avantageuse de l'invention illustrée à la figure 9, le procédé selon l'invention consiste à définir également une représentation tridimensionnelle numérisée R₄ₐ. de la surface interne de la coiffe définie à partir de la représentation numérisée R₃ de la surface externe de la chape en tenant compte des règles liées à l'insertion et au scellement d'une coiffe sur une chape. Ainsi, il peut être prévu de laisser subsister, notamment, un espace entre la représentation numérisée R₄ₐ. de la surface interne de la coiffe et la représentation numérisée R₃ de la surface externe de la chape pour la mise en place du ciment de scellement.

Le procédé selon l'invention consiste, ensuite, à définir une représentation tridimensionnelle numérisée R₄ de la surface externe de la coiffe définie à partir de la représentation numérisée R₂ de la surface externe de la prothèse et de la limite externe de la représentation numérisée R₄ₐ de la surface interne de la coiffe. Il est à noter que le volume compris entre la surface R₄ₐ. et la surface R₄ est représentatif du volume total de la coiffe. Bien entendu, les représentations numérisées R₄ₐ. et R₄ sont obtenues à l'aide de moyens de programmation appropriés.

Lorsque la représentation numérisée tridimensionnelle R₃ de la chape 3 a été choisie, il peut être procédé à un usinage d'une chape prothétique correspondante 3 (figure 10). Dans ce cas, le nuage de points correspondant à la représentation numérisée tridimensionnelle R₃ de la surface externe de la chape et le nuage de points correspondant à la représentation numérisée tridimensionnelle R_{1c}, de la surface interne de la prothèse sont délivrés à toute installation capable d'exploiter les informations numériques, telle qu'une machine à commande numérique. De même, il peut être procédé à un usinage d'une coiffe prothétique correspondante 4 (figure 11). Dans ce cas, le nuage de points correspondant à la représentation numérisée tridimensionnelle R₄ₐ. de la surface interne de la coiffe et le nuage de points correspondant à la représentation numérisée tridimensionnelle R₄ de la surface externe de la coiffe sont délivrés à toute installation capable d'exploiter les informations numériques

La définition de la chape 3, selon le procédé selon l'invention, permet d'optimiser sa réalisation, aussi bien du point de vue mécanique, qu'esthétique. La chape 3 peut ainsi présenter un profil interne anatomique respectant les informations cliniques, mais également un profil externe respectant les critères, notamment, de tenue mécanique et d'esthétique pour la réalisation de la coiffe 4. Dans le même sens et tel que cela ressort clairement de la figure 12, un tel procédé permet de compenser automatiquement les défauts de la zone d'implantation, en permettant de réaliser une représentation numérisée R₃ de la surface externe de la chape qui s'adapte à la morphologie de la zone d'implantation tout en conférant à la coiffe 4 une épaisseur constante. Un autre avantage du procédé selon l'invention est qu'il permet, soit de choisir les paramètres d'usinage en fonction des contraintes liées aux surfaces à usiner, soit d'adapter le profil des surfaces à usiner en fonction du mode d'usinage.

Dans l'exemple considéré ci-dessus, la prothèse est constituée de deux pièces distinctes, à savoir une chape 3 et une coiffe 4. Il est à noter que le procédé selon l'invention peut être mis en oeuvre pour une prothèse ne comportant pas deux pièces séparées. Ainsi, il peut être envisagé de déterminer une représentation tridimensionnelle numérisée R₃ de la surface externe de la chape, de façon qu'elle se trouve, au moins localement, sous-dimensionnée par rapport à la surface externe de la prothèse déterminée par la représentation tridimensionnelle numérisée R₂, La chape prothétique 3, réalisée à partir de cette représentation numérisée, présente donc un sous-dimensionnement au moins localisé permettant un apport de matière sur la chape. Cet apport de matière est constitutif de la coiffe et se trouve, en général, travaillé ou usiné, afin de conférer l'aspect esthétique définitif à la prothèse.

Dans une autre forme de mise en oeuvre de l'invention, il peut être prévu de déterminer une représentation tridimensionnelle numérisée R₃ de la surface externe de la chape, de façon qu'elle se trouve, au moins localement, surdimensionnée par rapport à la surface externe de la prothèse déterminée par la représentation tridimensionnelle numérisée R₂. La chape prothétique 3, réalisée à partir de cette représentation numérisée, possède une surépaisseur au moins localisée, qui est ensuite travaillée par enlèvement de matière pour constituer la coiffe prothétique définitive. Dans cet exemple de réalisation, la chape prothétique et la coiffe prothétique forment une pièce unique.

Les deux exemples de réalisation ci-dessus permettent une optimisation esthétique de la prothèse en offrant la possibilité d'un travail ou d'un usinage de la coiffe prothétique, constituée soit par un apport ou un enlèvement de matière. Bien entendu, il est clair que l'apport ou l'enlèvement de matière peuvent être mis en oeuvre simultanément pour la réalisation d'une même prothèse.

Dans la description qui précède, le procédé selon l'invention a été décrit en relation de la fabrication d'une prothèse concernant une unique dent. Il doit, bien entendu, être considéré que le procédé selon l'invention peut être mis en oeuvre pour une prothèse plurale. Dans ce cas, il est à noter que les différentes étapes du procédé selon l'invention peuvent être mises en oeuvre pour assurer la réalisation, les unes après les autres, des diverses dents de la prothèse plurale. De même, chaque étape du procédé peut être appliquée pour l'ensemble des dents de la prothèse.

L'invention n'est pas limitée aux exemples décrits et représentés, car diverses modifications peuvent y être apportées sans sortir de son cadre.

## Revendications

1. Procédé pour la réalisation d'une prothèse dentaire (2) comportant au moins, d'une part, une chape prothétique (3) destinée à être adaptée sur une zone d'implantation (5) du corps humain et, d'autre part, une coiffe prothétique (4) portée par la chape, *consistant à* :
- réaliser *par moulage une empreinte (6) de la zone d'implantation et établir à partir de l'empreinte moulée (6)* une représentation tridimensionnelle numérisée (R₁),
- définir une représentation tridimensionnelle (R_{1c}) de la surface interne de la prothèse, correspondant à la surface interne de la chape définie à partir de la représentation tridimensionnelle numérisée (R₁) de la zone d'implantation, en tenant compte des règles liées à l'insertion et au scellement d'une prothèse,
- définir une représentation tridimensionnelle numérisée (R₂) de la surface externe de la prothèse, à partir des paramètres environnementaux de la prothèse, en tenant compte des contraintes résultant de la surface interne (R_{1C}) de la prothèse, constituée par la surface interne de la chape,
***caractérisé en ce qu'**il consiste*
- à définir une représentation tridimensionnelle numérisée (R₃) de la surface externe de la chape, à partir de la surface externe (R₂) de la prothèse, en tenant compte de la surface interne (R_{1c}) de la prothèse, des critères de tenue mécanique, notamment d'épaisseur, et des critères esthétiques.

2. Procédé selon la revendication 1, **caractérisé en ce qu'**il consiste :
- à définir une représentation tridimensionnelle numérisée (R₄ₐ) de la surface interne de la coiffe définie à partir de la représentation tridimensionnelle numérisée (R₃) de la surface externe de la chape, en tenant compte des règles liées à l'insertion et au scellement d'une coiffe sur la chape,
- et à définir une représentation tridimensionnelle numérisée (R₄) de la surface externe de la coiffe définie à partir de la surface externe (R₂) de la prothèse, en tenant compte des limites externes de la surface interne (R₄ₐ) de la coiffe.

3. Procédé selon la revendication 1, **caractérisé en ce qu'**il consiste, à partir de la définition de la représentation tridimensionnelle numérisée (R₃) de la surface externe de la chape et de la représentation tridimensionnelle numérisée (R_{1c}) de la surface interne de la prothèse, à réaliser la chape prothétique (3) correspondante.

4. Procédé selon la revendication 1 ou 2, **caractérisé en ce qu'**il consiste, à partir de la définition de la représentation tridimensionnelle numérisée (R₄ₐ) de la surface interne de la coiffe et de la représentation tridimensionnelle numérisée (R₄) de la surface externe de la coiffe, à réaliser la coiffe prothétique (4) correspondante.

5. Procédé selon les revendications 1 à 4, **caractérisé en ce qu'**il consiste :
- à déterminer la représentation tridimensionnelle numérisée (R₃) de la surface externe de la chape, de façon qu'elle se trouve, au moins localement, sous-dimensionnée par rapport à la surface externe de la prothèse déterminée par la représentation tridimensionnelle numérisée (R₂),
- et, après la réalisation de la chape prothétique (3), à réaliser la coiffe prothétique (4) à l'aide d'un apport de matière déposé sur la chape prothétique (3).

6. Procédé selon les revendications 1 à 5, **caractérisé en ce qu'**il consiste :
- à déterminer la représentation tridimensionnelle numérisée (R₃) de la surface externe de la chape, de façon qu'elle se trouve, au moins localement, surdimensionnée par rapport à la surface externe de la prothèse déterminée par la représentation tridimensionnelle numérisée (R₂),
- et, après la réalisation de la chape prothétique (3), à réaliser la coiffe prothétique (4) par un enlèvement de matière de la chape prothétique (3).

7. Procédé selon l'une des revendications 1 à 6, **caractérisé en ce qu'**il consiste à réaliser une prothèse dentaire plurale comportant divers éléments constitutifs.

## Patentansprüche

1. Verfahren zur Herstellung einer Zahnprothese (2), welche aufweist: einerseits eine Prothesenkappe (3), die dazu ausgelegt ist, um an einen Implantationsbereich (5) des menschlichen Körpers angepasst zu werden, und andererseits eine Prothesenhülle (4), die von der Kappe getragen wird, wobei das Verfahren die folgenden Schritte aufweist:
- Herstellen eines Abdrucks (6) des Implantationsbereichs durch Giessen und Erstellen einer dreidimensionalen digitalisierten Darstellung (R₁) auf der Grundlage des gegossenen Abdrucks (6),
- Definieren einer dreidimensionalen Darstellung (R_{1c}) der inneren Oberfläche der Prothese, die der inneren Oberfläche der Kappe entspricht, die auf der Grundlage der dreidimensionalen digitalisierten Darstellung (R₁) des Implantationsbereichs definiert ist, indem man die Regeln in Verbindung mit dem Einfügen und dem Versiegeln einer Prothese berücksichtigt,
- Definieren einer dreidimensionalen digitalisierten Darstellung (R₂) der äußeren Oberfläche der Prothese auf der Grundlage von Umgebungsparametern der Prothese, indem man die Bedingungen berücksichtigt, die sich aus der inneren Oberfläche (R_{1c}) der Prothese ergeben und die durch die innere Oberfläche der Kappe gebildet werden,
**dadurch gekennzeichnet, dass** es einen Schritt aufweist zum
- Definieren einer dreidimensionalen digitalisierten Darstellung (R₃) der äußeren Oberfläche der Kappe auf der Grundlage der äußeren Oberfläche (R₂) der Prothese, indem man die innere Oberfläche (R_{1c}) der Prothese, Kriterien für den mechanischen Halt, insbesondere die Dicke, sowie ästhetische Kriterien berücksichtigt.

2. Verfahren nach Anspruch 1, **dadurch gekennzeichnet, dass** es die folgenden Schritte aufweist:
- Definieren einer dreidimensionalen digitalisierten Darstellung (R₄ₐ) der inneren Oberfläche der Hülle, die auf der Grundlage der dreidimensionalen digitalisierten Darstellung (R₃) der äußeren Oberfläche der Kappe definiert ist, indem man die Regeln in Verbindung mit dem Einfügen und dem Versiegeln einer Hülle auf der Kappe berücksichtigt, und
- Definieren einer dreidimensionalen digitalisierten Darstellung (R₄) der äußeren Oberfläche der Hülle, die auf der Grundlage der äußeren Oberfläche (R₂) der Prothese definiert ist, indem man die äußeren Grenzen der inneren Oberfläche (R₄ₐ) der Hülle berücksichtigt.

3. Verfahren nach Anspruch 1, **dadurch gekennzeichnet, dass** es einen Schritt aufweist zum Herstellen der entsprechenden Prothesenkappe (3) auf der Grundlage der Definition der dreidimensionalen digitalisierten Darstellung (R₃) der äußeren Oberfläche der Kappe und der dreidimensionalen digitalisierten Darstellung (R_{1c}) der inneren Oberfläche der Prothese.

4. Verfahren nach Anspruch 1 oder 2, **dadurch gekennzeichnet, dass** es einen Schritt aufweist zum Herstellen der entsprechenden Prothesenhülle (4) auf der Grundlage der Definition der dreidimensionalen digitalisierten Darstellung (R₄ₐ) der inneren Oberfläche der Hülle und der dreidimensionalen digitalisierten Darstellung (R₄) der äußeren Oberfläche der Hülle.

5. Verfahren nach einem der Ansprüche 1 bis 4, **dadurch gekennzeichnet, dass** es die folgenden Schritte aufweist:
- Bestimmen der dreidimensionalen digitalisierten Darstellung (R₃) der äußeren Oberfläche der Kappe derart, dass sie zumindest lokal unterdimensioniert ist bezüglich der äußeren Oberfläche der Prothese, die durch die dreidimensionale digitalisierte Darstellung (R₂) bestimmt ist, und
- Herstellen der Prothesenhülle (4) nach der Herstellung der Prothesenkappe (3) mit Hilfe einer Materialzufuhr, die auf die Prothesenkappe (3) aufgetragen wird.

6. Verfahren nach einem der Ansprüche 1 bis 5, **dadurch gekennzeichnet, dass** es die folgenden Schritte aufweist:
- Bestimmen der dreidimensionalen digitalisierten Darstellung (R₃) der äußeren Oberfläche der Kappe derart, dass sie zumindest lokal überdimensioniert ist bezüglich der äußeren Oberfläche der Prothese, die durch die dreidimensionale digitalisierte Darstellung (R₂) bestimmt ist, und
- Herstellen der Prothesenhülle (4) durch Entfernen von Materie von der Prothesenkappe (3) nach der Herstellung der Prothesenkappe (3).

7. Verfahren nach einem der Ansprüche 1 bis 6, **dadurch gekennzeichnet, dass** es einen Schritt aufweist zum Herstellen einer Mehrfach-Zahnprothese mit mehreren sie bildenden Elementen.

## Claims

1. Process for the production of a dental prosthesis (2) having at least, on the one hand, a prosthetic cap (3) intended to be fitted on an implantation zone (5) of the human body and, on the other hand, a prosthetic crown (4) supported by the cap, consisting in:
- taking an impression (6) of the implantation zone by moulding and, on the basis of the moulded impression (6), making a digitized three-dimensional representation (R₁),
- defining a three-dimensional representation (R_{1c}) of the internal surface of the prosthesis, corresponding to the internal surface of the cap, defined on the basis of the digitized three-dimensional representation (R₁) of the implantation zone, while taking account of the rules connected with the insertion and sealing of a prosthesis,
- defining a digitized three-dimensional representation (R₂) of the external surface of the prosthesis, on the basis of the environmental parameters of the prosthesis, while taking account of the constraints resulting from the internal surface (R_{1c}) of the prosthesis, consisting of the internal surface of the cap,
**characterized in that** it consists in defining a digitized three-dimensional representation (R₃) of the external surface of the cap, on the basis of the external surface (R₂) of the prosthesis, while taking account of the internal surface (R_{1c}) of the prosthesis, the criteria pertaining to mechanical durability, in particular thickness, and aesthetic criteria.

2. Process according to Claim 1, **characterized in that** it consists:
- in defining a digitized three-dimensional representation (R₄ₐ) of the internal surface of the crown, defined on the basis of the digitized three-dimensional representation (R₃) of the external surface of the cap, while taking account of the rules connected with the insertion and sealing of a crown on the cap,
- and in defining a digitized three-dimensional representation (R₄) of the external surface of the crown, defined on the basis of the external surface (R₂) of the prosthesis, while taking account of the external boundaries of the internal surface (R₄ₐ) of the crown.

3. Process according to Claim 1, **characterized in that** it consists, on the basis of the definition of the digitized three-dimensional representation (R₃) of the external surface of the cap and of the digitized three-dimensional representation (R_{1c}) of the internal surface of the prosthesis, in producing the corresponding prosthetic cap (3).

4. Process according to Claim 1 or 2, **characterized in that** it consists, on the basis of the definition of the digitized three-dimensional representation (R₄ₐ) of the internal surface of the crown and of the digitized three-dimensional representation (R₄) of the external surface of the crown, in producing the corresponding prosthetic crown (4).

5. Process according to Claims 1 to 4, **characterized in that** it consists:
- in determining the digitized three-dimensional representation (R₃) of the external surface of the cap, in such a way that it is at least locally under-dimensioned in relation to the external surface of the prosthesis determined by the digitized three-dimensional representation (R₂),
- and, after the production of the prosthetic cap (3), in producing the prosthetic crown (4) using addition of material deposited on the prosthetic cap (3).

6. Process according to Claims 1 to 5, **characterized in that** it consists:
- in determining the digitized three-dimensional representation (R₃) of the external surface of the cap, in such a way that it is at least locally over-dimensioned in relation to the external surface of the prosthesis determined by the digitized three-dimensional representation (R₂),
- and, after the production of the prosthetic cap (3), in producing the prosthetic crown (4) using removal of material from the prosthetic cap (3).

7. Process according to one of Claims 1 to 6, **characterized in that** it consists in producing a plural dental prosthesis having various constituent elements.
